# EUROPÄISCHE PATENTANMELDUNG

(11) **EP 3 804 701 A1**
(43) Veröffentlichungstag der Anmeldung: **14.04.2021**
(21) Anmeldenummer: 19202455.2
(22) Anmeldetag: 10.10.2019
(51) Int. Cl.: A61K 9/51, A61K 31/405, A61P 29/00

(54) **VERFAHREN ZUR HERSTELLUNG EINER PHARMAZEUTISCHEN FORMULIERUNG MIT KRISTALLINEN UND AMORPHEN ANTEILEN EINES WIRKSTOFFES**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Ein Verfahren zur Herstellung einer pharmazeutischen Formulierung umfasst die Schritte: A) Bereitstellen von Partikeln eines Polymers, wobei in die Partikel des Polymers weiterhin Partikel eines pharmazeutischen Wirkstoffes wenigstens teilweise eingebettet sind; B) Erwärmen der Partikel des Polymers auf eine vorbestimmte Temperatur für eine vorbestimmte Zeit und C) Abkühlen der Partikel des Polymers nach der vorbestimmten Zeit auf eine Temperatur von 18 °C bis 24 °C, wobei das Polymer wenigstens teilweise in Wasser löslich ist und der Wirkstoff in dem Polymer wenigstens teilweise löslich ist.

Der partikelförmige pharmazeutische Wirkstoff liegt in Form von Partikeln vor, deren d₉₀-Wert der Partikelgrößenverteilung ≤ 1 µm beträgt. Die vorbestimmte Temperatur liegt in einem Bereich von 20 °C unterhalb bis 30 °C oberhalb der Glasübergangstemperatur (bestimmt mit DSC gemäß DIN EN ISO 11357-2 bei einer Heizrate von 10 °C/min), maximal aber bis zur Schmelztemperatur des Wirkstoffs, des Polymers. Der Gesamtmengenanteil des Wirkstoffes in dem Polymer ist größer als als die Menge des bei der vorbestimmten Temperatur in dem Polymer löslichen Wirkstoffes.

Die Erfindung betrifft weiterhin eine pharmazeutische Formulierung, umfassend einen mit einem zumindest teilweise in Wasser löslichen Polymer beschichteten partikelförmigen pharmazeutischen Wirkstoff, ein Verfahren zur Herstellung einer Suspension einer pharmazeutischen Formulierung und eine Suspension eines pharmazeutischen Wirkstoffes.

## Beschreibung

Die vorliegende Erfindung betrifft ein Verfahren zur Herstellung einer pharmazeutischen Formulierung, umfassend die Schritte: A) Bereitstellen von Partikeln eines Polymers, wobei in die Partikel des Polymers weiterhin Partikel eines pharmazeutischen Wirkstoffes wenigstens teilweise eingebettet sind, B) Erwärmen der Partikel des Polymers auf eine vorbestimmte Temperatur für eine vorbestimmte Zeit und C) Abkühlen der Partikel des Polymers nach der vorbestimmten Zeit auf eine Temperatur von 18 °C bis 24 °C, wobei das Polymer wenigstens teilweise in Wasser löslich ist und der Wirkstoff in dem Polymer wenigstens teilweise löslich ist. Die Erfindung betrifft weiterhin eine pharmazeutische Formulierung, umfassend einen mit einem zumindest teilweise in Wasser löslichen Polymer beschichteten partikelförmigen pharmazeutischen Wirkstoff, ein Verfahren zur Herstellung einer Suspension einer pharmazeutischen Formulierung und eine Suspension eines pharmazeutischen Wirkstoffes.

Eine hohe Auflösungsrate eines pharmazeutischen Wirkstoffes resultiert meistens in einer erhöhten Bioverfügbarkeit, zumindest aber in einer verbesserten Kinetik der Bioverfügbarkeit. Dies kann beispielsweise durch die Vergrößerung der spezifischen Oberfläche des Wirkstoff-Partikelkollektivs realisiert werden. So weisen Wirkstoffnanosuspensionen eine wesentlich höhere Auflösungsrate als eine mikronisierte Suspension vor. Eine weitere Methode zur Erhöhung der Auflösungsrate und der Löslichkeit stellt die Herstellung von amorphen festen Wirkstoff-Polymer-Dispersionen dar. Dabei wird der Wirkstoff in einer Polymermatrix molekular dispergiert und so amorph stabilisiert. Dieses System ist nur dann thermodynamisch stabil, wenn das Polymer den vorhandenen Wirkstoff vollständig lösen kann. Resultierend aus niedrigen Löslichkeiten des Wirkstoffs im Polymer bei Raumtemperatur ist meist nur wenig Wirkstoff stabil in einer Polymermatrix löslich.

Wang et al. berichtet im Artikel "Stability of nanosuspensions in drug delivery", Journal of Controlled Release 172 (2013) 1126-1141, von durch Gefriertrocknung hergestellten redispergierbaren Nanopartikel enthaltenden Pulvern. Auch hier wurden neben den üblichen Stabilisatoren zusätzliche Gerüstbildner verwendet. SDS wird nur als Additiv für die Mahlung verwendet.

Der Übersichtsartikel "Polymeric Amorphous Solid Dispersions: A Review of Amorphization, Crystallization, Stabilization, Solid-State Characterization, and Aqueous Solubilization of Biopharmaceutical Classification System Class II Drugs," von S. Baghel et al., Journal of Pharmaceutical Sciences 105, 9, 2016, Seiten 2527-2544 berichtet von der Herstellung von amorphen festen Dispersionen. Dabei werden keine Hybrid-Systeme erwähnt.

Im Artikel "Polymorphism of Indomethacin in Semicrystalline Dispersions: Formation, Transformation, and Segregation" von Van Duong et al., Mol. Pharmaceutics 2018, 15, 1037-1051 wird über semi-kristalline Polymer-Wirkstoff-Systeme berichtet, wobei hier ausgehend von einer amorphen festen Dispersion durch verschiedene Wirkstoffgehalte unterschiedliche Polymorphe entstehen. Nachteilig an dieser Methode ist, dass kein kontrolliertes Wachstum vorliegt und somit unterschiedlich große Kristallite entstehen. Zusätzlich wird kristallines PEG verwendet. Zudem entstehen unterschiedliche Polymorphe. Die amorphe Phase ist am Ende nahezu vollständig kristallin.

Die vorliegende Erfindung hat sich die Aufgabe gestellt, verbesserte pharmazeutische Formulierungen bereitzustellen, welche eine höhere Wirkstoffbeladung bei gleichzeitig variabler Freisetzungskinetik ermöglichen.

Erfindungsgemäß gelöst wird diese Aufgabe durch ein Verfahren gemäß Anspruch 1, eine Formulierung gemäß Anspruch 11, ein Verfahren gemäß Anspruch 13 und eine Dispersion gemäß Anspruch 15. Vorteilhafte Weiterbildungen sind in den Unteransprüchen angegeben. Sie können beliebig kombiniert werden, sofern sich aus dem Kontext nicht eindeutig das Gegenteil ergibt.

Ein Verfahren zur Herstellung einer pharmazeutischen Formulierung umfasst die Schritte:
A) Bereitstellen von Partikeln eines Polymers, wobei in die Partikel des Polymers weiterhin Partikel eines pharmazeutischen Wirkstoffes wenigstens teilweise eingebettet sind;
B) Erwärmen der Partikel des Polymers auf eine vorbestimmte Temperatur für eine vorbestimmte Zeit;
C) Abkühlen der Partikel des Polymers nach der vorbestimmten Zeit auf eine Temperatur von 18 °C bis 24 °C;
wobei das Polymer wenigstens teilweise in Wasser löslich ist und der Wirkstoff in dem Polymer wenigstens teilweise löslich ist,
der partikelförmige pharmazeutische Wirkstoff in Form von Partikeln vorliegt, deren d₉₀-Wert der Partikelgrößenverteilung (volumenbasiert; bestimmt mittels Laserbeugung gemäß ISO 13320:2009) ≤ 1 µm beträgt, dass
die vorbestimmte Temperatur in einem Bereich von 20 °C unterhalb bis 30 °C oberhalb der Glasübergangstemperatur (bestimmt mit DSC gemäß DIN EN ISO 11357-2 bei einer Heizrate von 10 °C/min) des Polymers liegt, maximal aber unterhalb der Schmelztemperatur des Wirkstoffs (bestimmt mit DSC gemäß DIN EN ISO 11357-2 bei einer Heizrate von 10 °C/min), und
der Gesamtmengenanteil des Wirkstoffes in dem Polymer größer ist als die Menge des bei der vorbestimmten Temperatur in dem Polymer löslichen Wirkstoffes.

Die vorliegende Erfindung stellt ein pharmazeutisches Wirkstoffsystem bereit, was sowohl amorphen als auch nanopartikulären Wirkstoff enthält. Der partikuläre Anteil liegt dabei in Form von isolierten Nanopartikeln eingebettet in einer amorphen festen Lösung vor. Durch diese Kombination von amorphem und nanopartikulärem Anteil werden die jeweiligen Eigenschaften vereint. Zusätzlich liegen höhere Wirkstoffgehalte vor, als bei einer reinen amorphen festen Lösung. Zudem gibt es Vorteile in der Freisetzungskinetik, da zum einen feste Wirkstoff-Nanopartikeln (über die Partikelgröße einstellbare Lösegeschwindigkeit), und zum anderen eine amorphe feste Lösung mit dem dafür typischen "Spring & Parachute"-Effekt vorliegt.

In Schritt A) des Verfahrens werden Polymerpartikel bereitgestellt, in welche Partikel eines Wirkstoffes wenigstens teilweise eingebettet sind. Eine äquivalente Beschreibung ist, dass die Wirkstoffpartikel von dem Polymer wenigstens teilweise umhüllt sind. Das Polymer ist wenigstens teilweise wasserlöslich. Unter "wasserlöslich" ist hierbei zu verstehen, daß sich bei 20 °C in 100 g Wasser mindestens 0.5 g, bevorzugt mindestens 2 g des Polymers auflösen beziehungsweise unter Gelbildung lösen.

Das Polymer kann ein neutrales Polymer oder ein kationischer oder anionischer Polyelektrolyt sein und kann ausgewählt sein aus der Gruppe: Alkylcellulosen, Hydroxyalkylcellulosen, Hydroxyalkylalkylcellulosen, Carboxyalkylcellulosen, Alkalimetallsalze der Carboxyalkylcellulosen, Carboxyalkylalkylcellulosen, Carboxyalkylcelluloseester, Stärken, Pektine, Chitinderivate, Polysaccharide, Polyacrylsäure und ihre Salze, Polymethacrylsäure und ihre Salze, Polyvinylalkohol, Polyvinylpyrrolidon, Polyalkylenoxide, Copolymere aus den genannten Polymertypen oder eine Mischung aus mindestens zwei der vorgenannten Polymere.

Der Wirkstoff ist in dem Polymer wenigstens teilweise löslich. Vorzugsweise beträgt die Löslichkeit des Wirkstoffs in dem Polymer bei der vorbestimmten Temperatur mehr als 0.5 g Wirkstoff pro 100 g Polymer, mehr bevorzugt mehr als 2 g Wirkstoff pro 100 g Polymer. Bei Wirkstoffgemischen wird die am schlechtesten lösliche Komponente als Bezugsgröße verwendet.

Beispiele für geeignete Wirkstoffklassen sind Benzodiazepine, Antihypertensiva, Vitamine, Cytostatika - insbesondere Taxol, Anästhetika, Neuroleptika, Antidepressiva, antiviral wirksame Mittel wie beispielsweise Anti-HIV wirksame Mittel, Antibiotika, Antimykotika, Antidementiva, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel oder Mischungen aus mindestens zwei der vorgenannten Wirkstoffklassen.

Als Wirkstoffe kommen insbesondere in Wasser schwerlösliche Wirkstoffe in Betracht. Hierunter werden Wirkstoffe verstanden, welche eine Löslichkeit von höchstens 1 g, vorzugsweis höchstens 0.1 g und mehr bevorzugt höchstens 0.01 g in 100 g Wasser bei 20 °C aufweisen.

Hinsichtlich der Partikelgröße des Wirkstoffes ist bevorzugt, dass der d₉₀-Wert der Partikelgrößenverteilung (d₉₀ bedeutet im Zusammenhang mit der vorliegenden Erfindung, dass volumenbasiert 90% aller Partikel maximal diesen Durchmesser aufweisen; die Bestimmung erfolgt mittels Laserbeugung gemäß ISO 13320:2009) ≥ 10 nm bis ≤ 1 µm beträgt, bevorzugt ≥ 50 nm bis ≤ 500 nm und besonders bevorzugt ≥ 30 nm bis ≤ 300 nm. Vorzugsweise sind diese Wirkstoffpartikel wenigstens teilweise kristallin, mehr bevorzugt kristallin. Dann lässt sich auch von nanokristallinen Wirkstoffpartikeln sprechen.

Schritt B) ist ein Temperschritt, in dem für eine vorbestimmte Zeit auf -20 °C bis +30 °C der Glasübergangstemperatur des Polymers, maximal aber unterhalb der Schmelztemperatur des Wirkstoffs, erwärmt wird. Im Fall von Polymergemischen wird die niedrigste Glasübergangstemperatur der vorliegenden Komponenten als Bezugsgröße ausgewählt und im Fall von Wirkstoffgemischen die niedrigste Schmelztemperatur der vorliegenden Komponenten.. Vorzugsweise wird auf ± 10 °C, mehr bevorzugt ±5 °C der T_{g} erwärmt. Ohne auf eine Theorie festgelegt zu sein wird angenommen, dass das Polymer im Bereich um T_{g} eine erhöhte Mobilität zulässt, so dass die Löseeigenschaften des Polymers für den Wirkstoff, d.h. die Einbettung von Wirkstoff-Molekülen in der Polymermatrix, an Bedeutung gewinnen.

Damit nach dem Temperschritt noch partikulärer Wirkstoff vorliegt und nicht der gesamte Wirkstoff im Polymer gelöst ist, ist vorgesehen, dass der Gesamtmengenanteil des Wirkstoffes in dem Polymer größer ist als die Menge des bei der vorbestimmten Temperatur in dem Polymer lösbaren Wirkstoffes. Vorzugsweise ist der Wirkstoff bis zu seiner thermodynamischen Sättigung im Polymer dispergiert, besonders bevorzugt molekular dispergiert.

In einer Ausführungsform beträgt die vorbestimmte Zeit in Schritt B) ≥ 1 Sekunde bis ≤ 10 Stunden. Vorzugsweise beträgt die vorbestimmte Zeit ≥ 1 Minute bis ≤ 5 Stunden, mehr bevorzugt ≥ 1 Stunde bis ≤ 4 Stunden.

In einer weiteren Ausführungsform erfolgt das Bereitstellen in Schritt A) durch Mahlen einer Suspension umfassend Partikel des Wirkstoffes und eine wässrige Lösung des Polymers und anschließendem Trocknen. Über den Weg einer wässrigen Lösung des Polymers lassen sich weiterhin Tenside, insbesondere ionische Tenside, in das System einführen.

In einer weiteren Ausführungsform wird in Schritt B) eine Suspension umfassend Partikel des Wirkstoffes und eine wässrige Lösung des Polymers aus einer Düse einer Mehrstoffdüse verdüst und ein Gas der vorbestimmten Temperatur wird aus einer weiteren Düse der Mehrstoffdüse ausgetragen, so dass die Suspension getrocknet wird und das getrocknete Material auf die vorbestimmte Temperatur erwärmt wird. Bei der Herstellung des Hybridsystems aus der Wirkstoff-Nanosuspension mittels Sprühtrocknung (Zweistoffdüse) wird die Suspension vorzugsweise oberhalb der Glasübergangstemperatur des Polymers versprüht. Analog zur Herstellung aus dem Pulver löst sich auch hier ein Teil des Wirkstoffes im Polymer, wodurch ein amorph-kristallines Hybridsystem entsteht. Zusätzlich bleibt die kristalline Form erhalten.

In einer weiteren Ausführungsform wird in Schritt B) eine Suspension umfassend Partikel des Wirkstoffes aus einer Düse einer Mehrstoffdüse verdüst, eine wässrige Lösung des Polymers und des Wirkstoffes wird aus einer anderen Düse der Mehrstoffdüse verdüst, so dass eine Mischung mit der verdüsten Partikelsuspension eintritt und es wird weiterhin ein Gas der vorbestimmten Temperatur aus einer weiteren Düse der Mehrstoffdüse ausgetragen, so dass die Mischung getrocknet wird und das getrocknete Material auf die vorbestimmte Temperatur erwärmt wird. Bei der Herstellung des Hybridsystems aus der Wirkstoff-Nanosuspension und einer Wirkstoff-Polymer-Lösung mittels Sprühtrocknung (Dreistoffdüse) werden Suspension (bevorzugt wässrig) und Lösung zusammen bei Temperaturen unterhalb der Glasübergangstemperatur des Polymers versprüht. Dabei wird die Wirkstoff-Polymer-Lösung mittels für das Wirkstoff-Polymer System geeignete Lösungsmittel (z.B. Ethanol, Aceton) hergestellt. Der Wirkstoff und die Additive müssen vollständig gelöst vorliegen. Im festen Zustand liegt eine amorphe feste Lösung kombiniert mit kristallinen Nanopartikeln vor. Zusätzlich bleibt bevorzugt die kristalline Form erhalten.

In einer weiteren Ausführungsform ist der pharmazeutische Wirkstoff ausgewählt aus: Cyclosporin A, Cyclosprin G, Rapamycin, Tacrolimus, Deoxyspergualin, Mycophenolate-Mofetil, Gusperimus; Acetylsalicylsäure, Ibuprofen, S(+)-Ibuprofen, Indomethacin, Diclofenac, Piroxicam, Meloxicam, Tenoxicam, Naproxen, Ketoprofen, Flurbiprofen, Fenoprofen, Felbinac, Sulindac, Etodolac, Oxyphenbutazon, Phenylbutazon, Nabumeton; Nifedipin, Nitrendipin, Nimodipin, Nisoldipin, Isradipin, Felodipin, Amlodipin, Nilvadipin, Lacidipin, Benidipin, Masnidipin, Furnidipin, Niguldipin; α-Liponsäure; Muramyldipeptid oder -tripeptid, Romurtid; Vitamin A, D, E oder F; Vincopectin, Vincristin, Vinblastin, Reserpin, Codein; Bromocriptin, Dihydroergotamin, Dihydroergocristin; Chlorambucil, Etoposid, Teniposid, Idoxifen, Tallimustin, Teloxantron, Tirapazamin, Carzelesin, Dexniguldipin, Intoplicin, Idarubicin, Miltefosin, Trofosfamid, Teloxantrone, Melphalan, Lomustin, 4,5-Bis(4'fluoranilino)-phthalimid; 4,5-Dianilinophthalimid.; Thymoctonan, Prezatid-Kupferacetat; Erythromycin, Daunorubicin, Gramicidin, Doxorubicin, Amphotericin B, Gentamycin, Leucomycin, Streptomycin, Ganefromycin, Rifamexil, Ramoplanin, Spiramycin; Fluconazol, Ketoconazol, Itraconazol; Famotidin, Cimetidin, Ranitidin, Roxatidin, Nizatidin, Omeprazol; N-[4-Methyl-3-(4-pyridin-3- -ylpyrimidin-2-ylamino)-phenyl]-benzamid, N-Benzyol-staurosporin; BOC-PhecPhe-Val-Phe-Morpholin oder sein O-[2-(2-methoxyethoxy)-acetoxy]-Derivat; N-[4-(5-Cyclopentyloxy-carbonylamino-1-methylindol-3-ylmethyl)-3-methoxybenzoyl]-2-vinyloxy]-benzolsulfonamid oder einer Mischung aus mindestens zwei der vorgenannten Wirkstoffe.

In einer weiteren Ausführungsform ist das Polymer ausgewählt aus: Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Natriumcarboxymethylcellulose, Carboxymethylethylcellulose, Carboxyalkylcelluloseester, Stärken, Natriumcarboxymethylamylopektin, Chitosan, Alginsäure, Alkalimetall- und Ammoniumsalze der Alginsäure, Carrageenane, Galaktomannane, Traganth, Agar-Agar, Gummi arabicum, Guargummi, Xanthangummi, Polyacrylsäure und ihre Salze, Polymethacrylsäure und ihre Salze, Polyvinylalkohol, Polyvinylpyrrolidon, Polyethylenoxid, Polypropylenoxid, Copolymere aus Ethylenoxid und Propylenoxid, N-Vinylpyrrolidon-Vinylacetat-Copolymere oder eine Mischung aus mindestens zwei der vorgenannten Polymere. Besonders bevorzugt sind Polyvinylpyrrolidone (insbesondere K12 und K30-Typen) und N-Vinylpyrrolidon-Vinylacetat-Copolymere.

In einer weiteren Ausführungsform enthalten die Partikel des Polymers weiterhin ein ionisches Tensid. Das ionische Tensid kann ein anionisches, kationisches oder zwitterionisches (amphoteres) Tensid sein. Ohne auf eine Theorie festgelegt zu sein wird angenommen, dass das ionische Tensid sich in Kombination mit dem Polymer positiv auf die Stabilität der Wirkstoffpartikel während der Trocknung auswirkt. Die Partikel sind folglich durch die Kombination aus elektrostatischer und sterischer Stabilisierung nahezu vollständig redispergierbar. Zudem kann beobachtet werden, dass ein polymorpher Zustand der Partikel erhalten bleibt. Dieses lässt sich sowohl mittels Röntgenpulverdiffraktometrie als auch mit Fourier-transformierter Infrarot-Spektroskopie dokumentieren.

In den Polymer/Wirkstoffpartikeln in Schritt A) kann der Polymergehalt ≥ 0.1 bis ≤ 40 Gewichts-% und der Tensidgehalt ≥ 0.001 bis ≤ 10 Gewichts-%, jeweils bezogen auf das Gesamtgewicht der Suspension in Schritt A), betragen. Ein weiteres Beispiel für eine Dosierung ist ein Gewichtsverhältnis von Wirkstoff: Polymer : Tensid von ≥ 0.01 bis ≤ 5 : 1 : ≥ 0.001 bis ≤ 1.

In einer weiteren Ausführungsform ist das ionische Tensid ausgewählt aus:
Acylaminosäuren (und deren Salze), wie: Acylglutamate, beispielsweise Natriumacylglutamat, Di-TEA-palmitoylaspartat und Natriucaprylglutamat; Acylpeptide, beispielsweise Palmitoylhydrolysiertes Milchprotein, Natrium Cocoylhydrolysiertes Soja Protein und Natrium-/Kalium Cocoyl-hydrolysiertes Kollagen; Sarcosinate, beispielsweise Myristoyl Sarcosin, TEA-lauroyl Sarcosinat, Natriumlauroylsarcosinat und Natriumcocoylsarkosinat; Taurate, beispielsweise Natriumlauroyltaurat und Natriummethylcocoyltaurat; Acyllactylate, Luroyllactylat, Caproyllactylat, Alaninate; Carbonsäuren und Derivate, wie: Carbonsäuren, beispielsweise Laurinsäure, Aluminiumstearat, Magnesiumalkanolat und Zinkundecylenat, Ester-Carbonsäuren, beispielsweise Calciumstearoyllactylat und Natrium PEG-Lauramidcarboxylat, EtherCarbonsäuren, beispielsweise Natriumlaureth-Carboxylat und Natrium PEG-Cocamide Carboxylat; Phosphorsäureester und Salze, wie beispielsweise DEA-Oleth--Phosphat und Dilaureth-Phosphat; Sulfonsäuren und Salze, wie Acyl-isethionate, z.B. Natrium-/ Ammoniumcocoyl-isethionat, Alkylarylsulfonate, Alkylsulfonate, beispielsweise Natriumcocosmonoglyceridsulfat, Natrium C-Olefin-sulfonat, Natriumlaurylsulfoacetat und Magnesium PEG-Cocamidsulfat, Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat; sowie Schwefelsäureester, wie Alkylethersulfat, beispielsweise Natrium-, Ammonium-, Magnesium-, MIPA-, TIPA-Laurethsulfat, Natriummyrethsulfat und Natrium C-Parethsulfat, Alkylsulfate, beispielsweise Natrium-, Ammonium-und TEA-Laurylsulfat.

Erfindungsgemäß können das oder die ionischen Tenside ferner vorteilhaft gewählt werden aus der Gruppe der kationischen Tenside. Vorteilhaft zu verwendende kationische Tenside sind Alkylamine, Alkylimidazole, ethoxylierte Amine, quaternäre Tenside und Esterquats.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder - bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersultate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Erfindungsgemäß können das oder die ionischen Tenside vorteilhaft gewählt werden aus der Gruppe der amphoteren Tenside.

Vorteilhaft zu verwendende amphotere Tenside sind: Acyl-/dialkylethylendiamine, beispielsweise Natriumacylamphoacetat, Dinatriumacylamphodipropionat, Dinatriumalkylamphodiacetat, Natriumacylamphohydroxypropylsultonat, Dinatriumacylamphodiacetat und Natriumacylamphopropionat sowie N-Alkylaminosäuren, beispielsweise Aminopropylalkylglutamid, Alkylaminopropionsäure, Natuiumalkylimidodipropionat und Lauroamphocarboxyglycinat.

Besonders bevorzugt als Tensid ist Natriumdodecylsulfat (SDS), Natriumdocusat, Natriumoleat und/oder Natriumdesoxycholat.

In einer weiteren Ausführungsform liegen der Wirkstoff und das Polymer in einem Gewichtsverhältnis von ≥ 1:4 bis ≤ 9:1 (vorzugsweise ≥ 1:3 bis ≤ 3:1) zueinander vor.

In einer weiteren Ausführungsform liegen das Polymer und das Tensid in einem Gewichtsverhältnis von ≥ 10:1 bis ≤ 300:1 (vorzugsweise ≥ 20:1 bis ≤ 70:1) zueinander vor.

Ein weiterer Aspekt der Erfindung ist eine pharmazeutische Formulierung, umfassend einen mit einem zumindest teilweise in Wasser löslichen Polymer beschichteten partikelförmigen pharmazeutischen Wirkstoff, wobei der partikelförmige pharmazeutische Wirkstoff in Form von Partikeln vorliegt, deren d₉₀-Wert der Partikelgrößenverteilung (volumenbasiert; bestimmt mittels Laserbeugung gemäß ISO 13320:2009) ≤ 1 µm beträgt, in dem Polymer weiterhin der gleiche Wirkstoff auch in amorpher Form dispergiert vorliegt und der Gesamtmengenanteil des Wirkstoffes in dem Polymer größer ist als die Menge des bei 20 °C in dem Polymer löslichen Wirkstoffes.

Diese Formulierung kann durch ein erfindungsgemäßes Verfahren erhalten werden. Entsprechend sind die Definitionen und Ausführungsformen zum Verfahren, die vorstehend erläutert wurden, auch auf die Formulierung übertragbar. Das Vorliegen einer amorphen Dispersion des Wirkstoffs in dem Polymer lässt sich durch Röntgen-Pulverdiffraktometrie (XRPD; Cu-K_{α}-Strahlung) anhand der Abwesenheit von Reflexen für den kristallinen Wirkstoff erkennen. Speziell erwähnt sei die Ausführungsform erwähnt, dass das Polymer weiterhin ein ionisches Tensid enthält.

Die Erfindung betrifft weiterhin ein Verfahren zur Herstellung einer Suspension einer pharmazeutischen Formulierung, umfassend den Schritt der Suspension einer erfindungsgemäßen Formulierung in einem Suspensionsmedium. Der Gehalt an Wirkstoff kann ≥ 50 Gewichts-%, vorzugsweise ≥ 60 Gewichts-%, bezogen auf das Gesamtgewicht der Suspension, betragen. Vorzugsweise ist das Suspensionsmedium ein wässriges Suspensionsmedium. Es ist weiter bevorzugt, dass Wasser ohne weitere Zusätze verwendet wird.

Die Erfindung betrifft ebenfalls eine Suspension eines pharmazeutischen Wirkstoffes, erhältlich durch ein erfindungsgemäßes Verfahren.

### Beispiele

Die vorliegende Erfindung wird anhand der nachfolgenden Beispiele und Figuren näher erläutert, ohne jedoch darauf beschränkt zu sein. Die Abkürzung "wt%" bedeutet Gewichtsprozent und sind auf das Gesamtgewicht der wässrigen Suspension bezogen. PVP K12 ist ein Polyvinylpyrrolidon mit einem K-Wert (nach Fikentscher, DIN EN ISO 1628-1) von 12. SDS ist Natriumdodecylsulfat. KVA 64 ist Kollidon® VA64, ein Vinylpyrrolidon-Vinylacetat-Copolymer. Instrumentelle Analytik wurde mittels Fouriertransform-Infrarotspektroskopie (FTIR) und Röntgenpulverdiffraktometrie (XRPD) durchgeführt.

Die Glasübergangstemperaturen T_{g} von PVP K12 und KVA 64 wurden mittels dynamischer Differenzkalorimetrie (differential scanning calorimetry, DSC) gemäß DIN EN ISO 11357-2 bei einer Heizrate von 10 °C/min ermittelt. Für PVP K12 beträgt die T_{g} 107 °C und für KVA 64 101 °C.

### Beispiel 1: Indomethacin-PVP K12-System

Die Nanosuspension wurde mittels Planetenkugelmühle (Fritsch Pulverisette 5) hergestellt. Dazu wurden 10 wt% Indomethacin mit 6 wt% PVP K12 und 0,1 wt% SDS stabilisiert. Die Polymer-Tensid-Lösungen wurden separat hergestellt und gelöst. Anschließend wurde die Lösung mit Indomethacin-Pulver versetzt und die entstandene Suspension auf einer Rührplatte homogenisiert. Die Mahlräume wurden zu 60% (Volumen) mit 0,4-0,6 mm (SiLibeads, Zirkonoxid, Yttrium stabilisiert) Mahlkörpern und das Restvolumen mit Suspension, blasenfrei befüllt. Nach 1 h 30 min Mahldauer bei 400 rpm lag eine Nanosuspension mit Partikeln d₉₀ < 500 nm (Malvern, Mastersizer 2000) vor, die zur Trocknung verwendet werden konnte.

Zur Gefriertrocknung wurden 3 ml Vials mit 0,7 g Suspension (Füllstand < 1 cm) befüllt und in den auf -40°C vorgekühlten Gefriertrockner gestellt und getrocknet. Die resultierenden Wirkstoff-Nanopartikelenthaltenden Pulver wurden dann zur Herstellung der Hybridsysteme verwendet.

Die Wirkstoff-Nanopartikel enthaltenden Pulver (Wirkstoffgehalt > 60 wt% Wirkstoff, PVP K12, SDS) wurden dann bei ca. 100 °C für bis zu 4h ausgeheizt. Resultat waren amorph-kristalline Hybrid-Systeme, die sowohl aus amorpher fester Lösung, als auch fein dispergierter kristalliner Phase bestanden. Durch XRPD und FTIR-Messungen konnten die Misch-Systeme nachgewiesen werden.

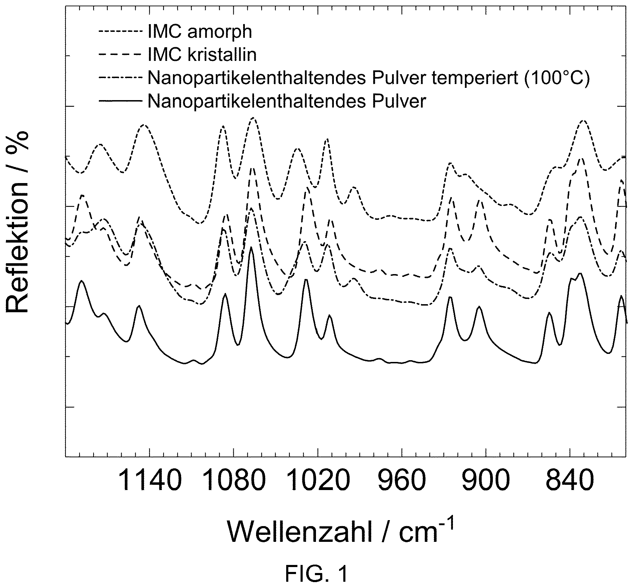

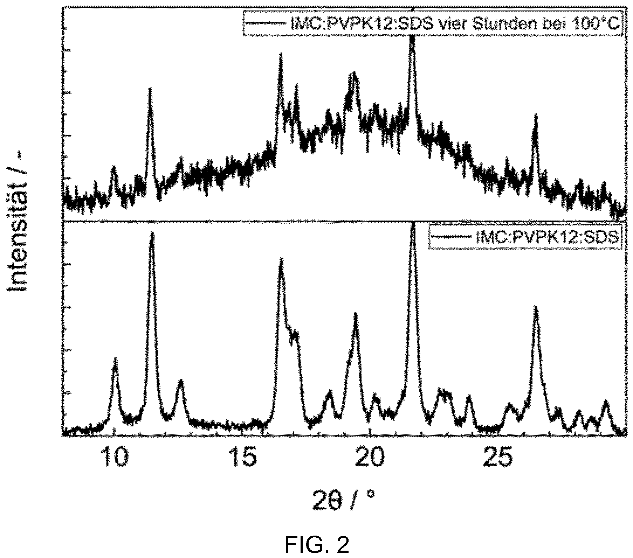

FIG. 1 zeigt FTIR-Spektren von amorphem und kristallinem Indomethacin (IMC), dem temperierten Indomethacin-Nanopartikelenthaltendem Pulver (IMC:PVP K12:SDS) und dem nicht temperierten Nanopartikelenthaltendem Pulver. FIG. 2 zeigt XRP-Diffraktogramme von temperiertem Indomethacin-Nanopartikel enthaltendem Pulver und nicht temperiertem Indomethacin-Nanopartikel enthaltendem Pulver.

Betrachtet man die FTIR-Spektren des rein amorphen und rein kristallinen Wirkstoffes, so wird deutlich, dass ein Peak bei 994 cm⁻¹ charakteristisch für amorphes Indomethacin und ein Peak bei 904 cm⁻¹ charakteristisch für kristallines Indomethacin ist.

Vergleicht man die temperierte Probe mit der nicht temperierten Probe, erkennt man in in FIG. 1, dass sowohl amorphe als auch kristalline Anteile nach der Temperierung vorliegen. Auch in FIG. 2 erkennt man, dass sowohl amorphe als auch kristalline Anteile in der temperierten Probe vorliegen, wobei die kristalline Form nicht beeinflusst wurde.

### Beispiel 2: Indomethacin-KVA 64-System

Die Nanopartikel enthaltenden Pulver wurden analog zu Beispiel 1 hergestellt, mit dem Unterschied, dass anstatt PVP K12 KVA 64 verwendet wurde.

Die Wirkstoff-Nanopartikel enthaltenden Pulver (Wirkstoffgehalt > 60 wt% Wirkstoff, KVA 64 (< 40 wt%), SDS) wurden dann bei ca. 100 °C ausgeheizt. Resultat waren amorph-kristalline Hybrid-Systeme die sowohl aus amorpher fester Lösung, als auch fein dispergierter kristalliner Phase bestanden. Durch XRPD und FTIR-Messungen konnten die Misch-Systeme nachgewiesen werden.

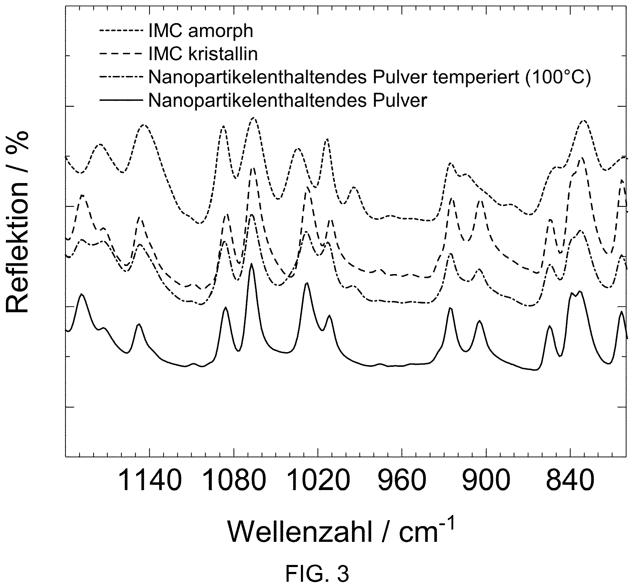

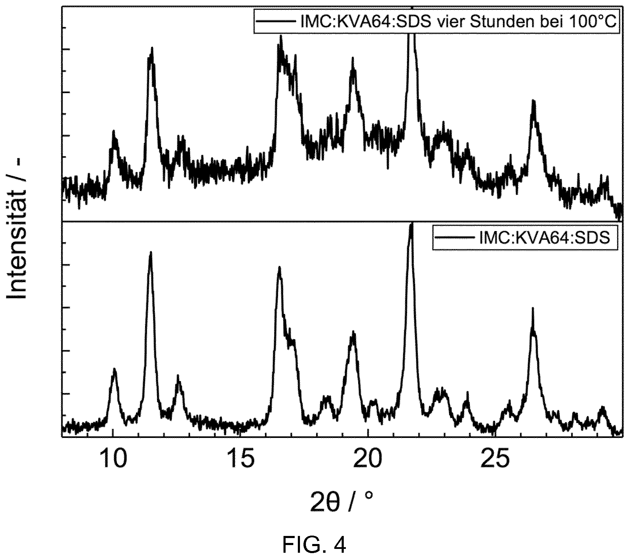

FIG. 3 zeigt FTIR-Spektren von amorphem und kristallinem Indomethacin (IMC), dem temperierten Indomethacin-Nanopartikelenthaltendem Pulver (IMC:KVA 64:SDS) und dem nicht temperierten Nanopartikelenthaltendem Pulver. FIG. 4 zeigt XRP-Diffraktogramme von temperiertem Indomethacin-Nanopartikel enthaltendem Pulver und nicht temperiertem Indomethacin-Nanopartikel enthaltendem Pulver.

Betrachtet man die FTIR-Spektren des rein amorphen und rein kristallinen Wirkstoffes, so wird deutlich, dass ein Peak bei 994 cm^-1 charakteristisch für amorphes Indomethacin und ein Peak bei 904 cm^-1 charakteristisch für kristallines Indomethacin ist. Vergleicht man nun die temperierte Probe mit der nicht temperierten Probe, erkennt man in FIG. 3, dass sowohl amorphe als auch kristalline Anteile nach der Temperierung vorliegen. Auch in FIG. 4 erkennt man, dass sowohl amorphe als auch kristalline Anteile in der temperierten Probe vorliegen, wobei die kristalline Form nicht beeinflusst wurde.

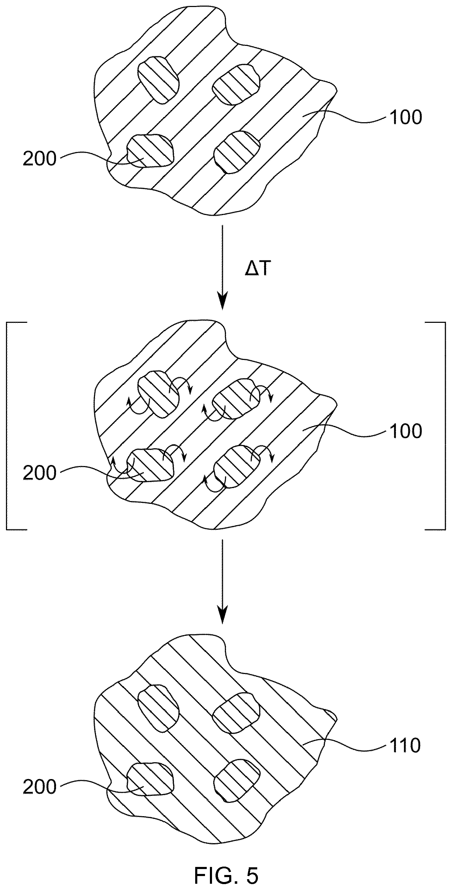

FIG. 5 zeigt schematisch das erfindungsgemäße Verfahren, in dem Partikel 100 eines zumindest teilweise in Wasser löslichen Polymers wie PVP K12 oder KVA 64 bereitgestellt werden, in dem der pharmazeutische Wirkstoff in Form einer Mehrzahl von nanokristallinen Partikel 200 vorliegt. Als Wirkstoff kommt insbesondere Indomethacin oder Naproxen in Frage.

Durch Temperaturerhöhung ΔT in die Nähe der Glasübergangstemperatur T_{g} des Polymers kommt es zu einem teilweisen Auflösen des Wirkstoffes in dem Polymer. Dieses ist durch die gebogenen Pfeile an den Partikel 200 im mittleren Bild symbolisiert.

Das unterste Bild stellt die erfindungsgemäße pharmazeutische Formulierung dar, die durch das Verfahren erhältlich ist. Neben den Wirkstoff-Nanopartikeln 200 liegt in dem Polymer 110 gelöster Wirkstoff vor. Dieses lässt sich als monomolekulare Vereinzelung des Wirkstoffes oder "solid dispersion" charakterisieren.

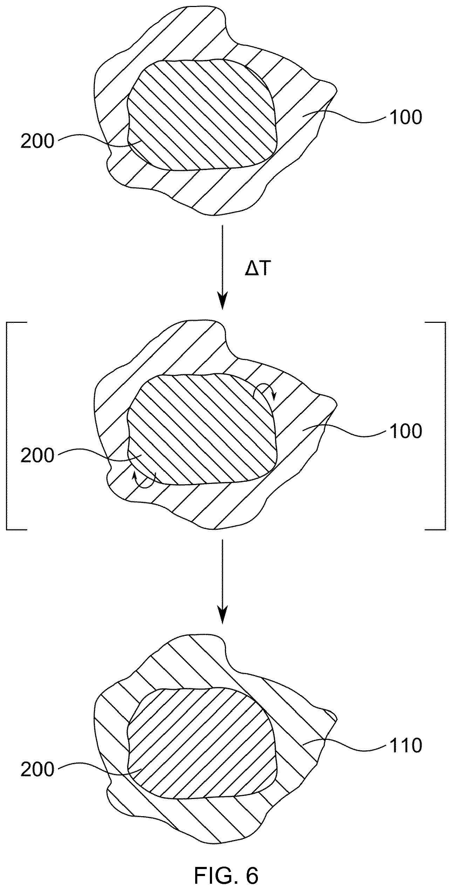

FIG. 6 variiert die Darstellung in FIG. 5 dahingehend, dass in dem Polymer 100, 110 nur ein einzelnes Wirkstoff-Nanopartikel vorliegt. Insofern müssen die Maßstäbe in FIG. 5 und 6 nicht die gleichen sein.

## Patentansprüche

1. Verfahren zur Herstellung einer pharmazeutischen Formulierung,
umfassend die Schritte:
A) Bereitstellen von Partikeln eines Polymers, wobei in die Partikel des Polymers weiterhin Partikel eines pharmazeutischen Wirkstoffes wenigstens teilweise eingebettet sind;
B) Erwärmen der Partikel des Polymers auf eine vorbestimmte Temperatur für eine vorbestimmte Zeit;
C) Abkühlen der Partikel des Polymers nach der vorbestimmten Zeit auf eine Temperatur von 18 °C bis 24 °C;
wobei das Polymer wenigstens teilweise in Wasser löslich ist und der Wirkstoff in dem Polymer wenigstens teilweise löslich ist,
**dadurch gekennzeichnet, dass**
der partikelförmige pharmazeutische Wirkstoff in Form von Partikeln vorliegt, deren d₉₀-Wert der Partikelgrößenverteilung (volumenbasiert; bestimmt mittels Laserbeugung gemäß ISO 13320:2009) ≤ 1 µm beträgt, dass
die vorbestimmte Temperatur in einem Bereich von 20 °C unterhalb bis 30 °C oberhalb der Glasübergangstemperatur (bestimmt mit DSC gemäß DIN EN ISO 11357-2 bei einer Heizrate von 10 °C/min) des Polymers liegt, maximal aber unterhalb der Schmelztemperatur des Wirkstoffs (bestimmt mit DSC gemäß DIN EN ISO 11357-2 bei einer Heizrate von 10 °C/min), und dass
der Gesamtmengenanteil des Wirkstoffes in dem Polymer größer ist als als die Menge des bei der vorbestimmten Temperatur in dem Polymer löslichen Wirkstoffes.

2. Verfahren gemäß Anspruch 1, wobei die vorbestimmte Zeit in Schritt B) ≥ 1 Sekunde bis ≤ 10 Stunden beträgt.

3. Verfahren gemäß Anspruch 1 oder 2, wobei das Bereitstellen in Schritt A) durch Mahlen einer Suspension umfassend Partikel des Wirkstoffes und eine wässrige Lösung des Polymers und anschließendem Trocknen erfolgt.

4. Verfahren gemäß einem der Ansprüche 1 bis 3, wobei in Schritt B) eine Suspension umfassend Partikel des Wirkstoffes und eine wässrige Lösung des Polymers aus einer Düse einer Mehrstoffdüse verdüst wird und ein Gas der vorbestimmten Temperatur aus einer weiteren Düse der Mehrstoffdüse ausgetragen wird, so dass die Suspension getrocknet wird und das getrocknete Material auf die vorbestimmte Temperatur erwärmt wird.

5. Verfahren gemäß einem der Ansprüche 1 bis 4, wobei in Schritt B) eine Suspension umfassend Partikel des Wirkstoffes aus einer Düse einer Mehrstoffdüse verdüst wird, eine wässrige Lösung des Polymers und des Wirkstoffes aus einer anderen Düse der Mehrstoffdüse verdüst wird, so dass eine Mischung mit der verdüsten Partikelsuspension eintritt und weiterhin ein Gas der vorbestimmten Temperatur aus einer weiteren Düse der Mehrstoffdüse ausgetragen wird, so dass die Mischung getrocknet wird und das getrocknete Material auf die vorbestimmte Temperatur erwärmt wird.

6. Verfahren gemäß einem der Ansprüche 1 bis 5, wobei der pharmazeutische Wirkstoff ausgewählt ist aus: Cyclosporin A, Cyclosprin G, Rapamycin, Tacrolimus, Deoxyspergualin, Mycophenolate-Mofetil, Gusperimus; Acetylsalicylsäure, Ibuprofen, S(+)-Ibuprofen, Indomethacin, Diclofenac, Piroxicam, Meloxicam, Tenoxicam, Naproxen, Ketoprofen, Flurbiprofen, Fenoprofen, Felbinac, Sulindac, Etodolac, Oxyphenbutazon, Phenylbutazon, Nabumeton; Nifedipin, Nitrendipin, Nimodipin, Nisoldipin, Isradipin, Felodipin, Amlodipin, Nilvadipin, Lacidipin, Benidipin, Masnidipin, Furnidipin, Niguldipin; α-Liponsäure; Muramyldipeptid oder -tripeptid, Romurtid; Vitamin A, D, E oder F; Vincopectin, Vincristin, Vinblastin, Reserpin, Codein; Bromocriptin, Dihydroergotamin, Dihydroergocristin; Chlorambucil, Etoposid, Teniposid, Idoxifen, Tallimustin, Teloxantron, Tirapazamin, Carzelesin, Dexniguldipin, Intoplicin, Idarubicin, Miltefosin, Trofosfamid, Teloxantrone, Melphalan, Lomustin, 4,5-Bis(4'fluoranilino)-phthalimid; 4,5-Dianilinophthalimid.; Thymoctonan, Prezatid-Kupferacetat; Erythromycin, Daunorubicin, Gramicidin, Doxorubicin, Amphotericin B, Gentamycin, Leucomycin, Streptomycin, Ganefromycin, Rifamexil, Ramoplanin, Spiramycin; Fluconazol, Ketoconazol, Itraconazol; Famotidin, Cimetidin, Ranitidin, Roxatidin, Nizatidin, Omeprazol; N-[4-Methyl-3-(4-pyridin-3-ylpyrimidin-2-ylamino)-phenyl]-benzamid, N-Benzyol-staurosporin; BOC-PhecPhe-Val-Phe-Morpholin oder sein O-[2-(2-methoxyethoxy)-acetoxy]-Derivat; N-[4-(5-Cyclopentyloxy-carbonylamino-1-methylindol-3-ylmethyl)-3-methoxybenzoyl]-2-vinyloxy]-benzolsulfonamid oder einer Mischung aus mindestens zwei der vorgenannten Wirkstoffe.

7. Verfahren gemäß einem der Ansprüche 1 bis 6, wobei das Polymer ausgewählt ist aus Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Natriumcarboxymethylcellulose, Carboxymethylethylcellulose, Carboxyalkylcelluloseester, Stärken, Natriumcarboxymethylamylopektin, Chitosan, Alginsäure, Alkalimetall- und Ammoniumsalze der Alginsäure, Carrageenane, Galaktomannane, Traganth, Agar-Agar, Gummi arabicum, Guargummi, Xanthangummi, Polyacrylsäure und ihre Salze, Polymethacrylsäure und ihre Salze, Polyvinylalkohol, Polyvinylpyrrolidon, Polyethylenoxid, Polypropylenoxid, Copolymere aus Ethylenoxid und Propylenoxid, N-Vinylpyrrolidon-Vinylacetat-Copolymere oder eine Mischung aus mindestens zwei der vorgenannten Polymere.

8. Verfahren gemäß einem der Ansprüche 1 bis 7, wobei die Partikel des Polymers weiterhin ein ionisches Tensid enthalten.

9. Verfahren gemäß einem der Ansprüche 1 bis 8, wobei der Wirkstoff und das Polymer in einem Gewichtsverhältnis von ≥ 1:4 bis ≤ 9:1 zueinander vorliegen.

10. Verfahren gemäß einem der Ansprüche 7 bis 9, wobei das Polymer und das Tensid in einem Gewichtsverhältnis von ≥ 10:1 bis ≤ 300:1 zueinander vorliegen.

11. Pharmazeutische Formulierung, umfassend einen mit einem zumindest teilweise in Wasser löslichen Polymer (110) beschichteten partikelförmigen pharmazeutischen Wirkstoff,
**dadurch gekennzeichnet, dass**
der partikelförmige pharmazeutische Wirkstoff in Form von Partikeln (200) vorliegt, deren d₉₀-Wert der Partikelgrößenverteilung ≤ 1 µm beträgt, dass
in dem Polymer (110) weiterhin der gleiche Wirkstoff auch in amorpher Form dispergiert vorliegt und dass
der Gesamtmengenanteil des Wirkstoffes in dem Polymer (110) größer ist als die Menge des bei 20 °C in dem Polymer (110) löslichen Wirkstoffes.

12. Formulierung gemäß Anspruch 11, wobei das Polymer (110) weiterhin ein ionisches Tensid enthält.

13. Verfahren zur Herstellung einer Suspension einer pharmazeutischen Formulierung, umfassend den Schritt der Suspension einer Formulierung gemäß Anspruch 11 oder 12 in einem Suspensionsmedium.

14. Verfahren gemäß Anspruch 13, wobei das Suspensionsmedium ein wässriges Suspensionsmedium ist.

15. Suspension eines pharmazeutischen Wirkstoffes, erhältlich durch ein Verfahren gemäß Anspruch 13 oder 14.
